(19) Europäisches Patentamt
European Patent Office
Office européen des brevets

(11) Numéro de publication: **0 227 539 B1**

# (12) FASCICULE DE BREVET EUROPEEN

(45) Date de publication du fascicule du brevet: **23.05.90**

(51) Int. Cl.⁵: **C07D 487/04**, A61K 31/55 // (C07D487/04, 249:00, 243:00)

(21) Numéro de dépôt: **86402763.6**

(22) Date de dépôt: **11.12.86**

(54) **Nouvelles 4H-triazolo /4,3-a/ /1,4/ benzodiazépines, leur procédé de préparation leur application comme médicaments et les compositions les renfermant.**

(30) Priorité: **13.12.85 FR 8518481**

(43) Date de publication de la demande: **01.07.87 Bulletin 87/27**

(45) Mention de la délivrance du brevet: **23.05.90 Bulletin 90/21**

(84) Etats contractants désignés: **CH DE ES GB IT LI NL**

(56) Documents cités: **FR-A- 2 137 714**

(73) Titulaire: **ROUSSEL-UCLAF, 35, boulevard des Invalides, F-75007 Paris(FR)**

(72) Inventeur: **Clemence, François, 2, rue Turgot, F-75009 Paris(FR)**
Inventeur: **Le Martret, Odile, 42, avenue de Versailles, F-75016 Paris(FR)**
Inventeur: **Delevallee, Françoise, 48-50, Avenue de la Dame Blanche, F-94120 Fontenay-Sous-Bois(FR)**

(74) Mandataire: **Bourgouin, André, Département des Brevets ROUSSEL UCLAF B.P. no 9, F-93230 Romainville(FR)**

EP 0 227 539 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles 4H-triazolo [4,3-a] [1,4] benzodiazépines, leur procédé de préparation, leur application comme médicaments et les compositions les renfermant.

Dans le brevet français BF 2 137 714 sont décrites des 4H-s-triazolo (4,3-a) benzodiazépines.

L'invention a pour objet les composés de formule (I) :

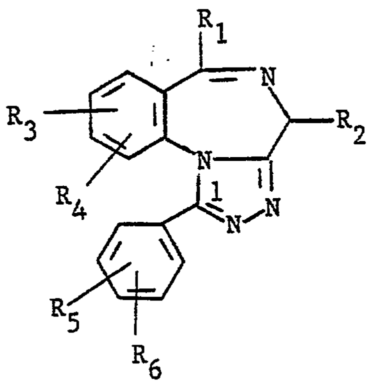

(I)

dans laquelle $R_1$ et $R_2$ représentent un atome d'hydrogène ou un radical alcoyle renfermant de 1 à 5 atomes de carbone, $R_3$, $R_4$, $R_5$, $R_6$, identiques ou différents, en position quelconque sur les noyaux benzéniques, représentent un atome d'hydrogène, un radical hydroxyle, un radical alcoyle renfermant de 1 à 4 atomes de carbone, un radical Oalc$_1$, alc$_1$ représentant un radical alcoyle renfermant de 1 à 4 atomes de carbone, un radical $NH_2$, NHalc$_2$, alc$_2$ représentant un radical alcoyle renfermant de 1 à 4 atomes de carbone, un radical

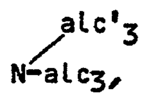

alc$_3$ et alc'$_3$, identiques ou différents, représentant un radical alcoyle renfermant de 1 à 4 atomes de carbone, un atome d'halogène, un radical $CF_3$ ou un radical $NO_2$, à la condition que $R_5$ et $R_6$ ne représentent pas à la fois un atome d'hydrogène, sous toutes les formes racémiques ou optiquement actives, ainsi que leurs sels d'addition avec les acides.

Lorsque $R_1$, $R_2$, $R_3$, $R_4$, $R_5$ et $R_6$ représentent un radical alcoyle, il s'agit de préférence du radical méthyle, éthyle, n-propyle, isopropyle ou n-butyle. Lorsqu'ils représentent un atome d'halogène, il s'agit de préférence d'un atome de chlore. Alc$_1$, alc$_2$, alc$_3$, alc'$_3$ représentent de préférence un radical méthyle, éthyle, n-propyle, isopropyle ou n-butyle.

Parmi les sels d'addition avec les acides, on peut citer ceux formés avec les acides minéraux tels que les acides chlorhydrique, bromhydrique, sulfurique ou phosphorique, ou les acides organiques tels que les acides acétique, propionique, oxalique, maléique, hémisuccinique ou trifluoroacétique.

L'invention concerne notamment les composés de formule (I) pour lesquels $R_1$ et $R_2$ représentent un radical méthyle, $R_3$ et $R_4$ représentent un atome d'hydrogène, et soit $R_5$ représente un atome d'hydrogène et $R_6$ représente un radical méthoxy, un atome d'halogène ou un radical $CF_3$, soit $R_5$ et $R_6$, identiques, représentent chacun un radical méthoxy, un atome d'halogène ou un radical $CF_3$, sous leurs formes racémiques ou optiquement actives, ainsi que leurs sels d'addition avec les acides.

L'invention a tout particulièrement pour objet la 4,6-diméthyl 1-(4-méthoxyphényl) 4H-1,2,4-triazolo [4,3-a] [1,4] benzodiazépine, ainsi que la 4,6-diméthyl 1-(4-chlorophényl 4H-1,2,4-triazolo [4,3-a] [1,4] benzodiazépine, sous leurs formes racémiques ou optiquement actives, ainsi que leurs sels d'addition avec les acides.

L'invention a aussi pour objet un procédé de préparation des produits de formule (I) tels que définis cidessus, caractérisé en ce que l'on soumet un composé de formule II :

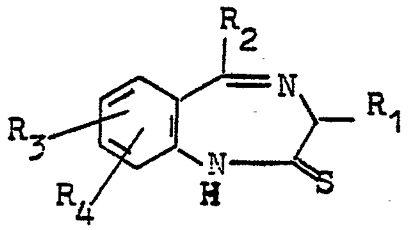

(II)

dans laquelle $R_1$, $R_2$, $R_3$ et $R_4$ ont les significations précédentes, sous forme racémique ou optiquement active, à l'action d'un composé de formule (III) :

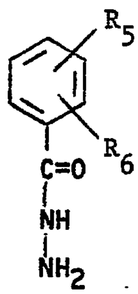

(III)

dans laquelle $R_5$ et $R_6$ ont les significations précédentes, pour obtenir un composé de formule (I) sous forme racémique ou optiquement active, que l'on transforme, si désiré, en sels par action d'un acide.

Dans un mode de réalisation préférée du procédé de l'invention, la réaction décrite ci-dessus s'effectue au reflux d'un solvant tel que le toluène, le tétrahydrofuranne, le chlorure de méthylène, les alcools.

Les composés de formule (I) tels que définis ci-dessus ainsi que leurs sels d'addition avec les acides présentent d'intéressantes propriétés pharmacologiques. Ils présentent, en particulier, une très bonne activité analgésique et de plus, ils sont actifs dans les modèles d'inflammation aiguë et chronique.

Ces propriétés justifient leur application en thérapeutique et l'invention a également pour objet à titre de médicaments, les produits tels que définis par la formule (I) ci-dessus, ainsi que les sels d'addition avec les acides pharmaceutiquement acceptables desdits produits de formule (I).

L'invention a plus particulièrement pour objet, à titre de médicaments, la 4,6-diméthyl 1-(4-méthoxyphényl) 4H-1,2,4-triazolo [4,3-a] [1,4] benzodiazépine, ainsi que la 4,6-diméthyl 1-(4-chlorophényl) 4H-1,2,4-triazolo [4,3-a] [1,4] benzodiazépine, sous leurs formes racémiques ou optiquement actives, ainsi que leurs sels d'addition avec les acides pharmaceutiquement acceptables.

Les médicaments, objets de l'invention, peuvent être utilisés dans le traitement des algies diverses d'origine musculaire, articulaire ou nerveuse, des douleurs dentaires, des migraines, du zona, également, à titre de traitement complémentaire, dans le traitement des états infectieux et fébriles.

Les médicaments, objets de la présente invention, peuvent encore être préconisés dans le traitement des maladies inflammatoires dégénératives telles que l'ostéoarthrose, collagénoses diverses (tendinites, etc ...), des maladies rhumatismales (la polyarthrite rhumatoïde, spondylarthrite enkylosante), ainsi que dans le traitement d'autres maladies de nature auto-immune telles que le lupus érythémateux disséminé, les glomérulonéphrites, la sclérose en plaques.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif par jour, par voie orale.

Les composés de formule (II), utilisés comme produits de départ, sont préparés comme indiqué dans le brevet français n° 2 137 714 par chauffage d'un composé de formule (IV) :

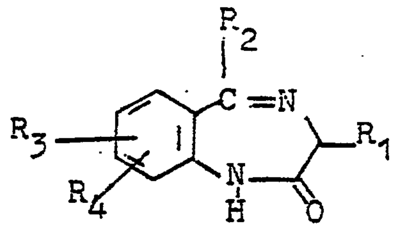

(IV)

dans laquelle $R_1$, $R_2$, $R_3$ et $R_4$ ont les significations précédentes, avec du pentasulfure de phosphore.
Les exemples donnés ci-après illustrent l'invention sans toutefois la limiter.

Exemple 1 : 4,6-diméthyl 1-(4-méthoxyphényl) 4H-1,2,4-triazolo [4,3-a] [1,4] benzodiazépine.

On porte au reflux pendant 15 heures, 8,43 g de 1,3-dihydro 2-thioxo 3,5-diméthyl 2H-1,4-benzodiazépine (3S) et 7,5 g d'hydrazide de l'acide p-méthoxy benzoïque dans 150 cm3 de toluène. On refroidit à 20°C, sèche, élimine les solvants sous pression réduite. On obtient 18,01 g d'huile brune que l'on chromatographie sur silice (éluant : chlorure de méthylène-méthanol 95-5). On amène à sec les fractions ainsi sélectionnées et obtient 8,12 g de gomme que l'on reprend dans l'éther éthylique et empâte au reflux pour concrétion de la gomme. On essore à 20°C, rince à l'éther, empâte dans l'éther et obtient 6,53 g de produit que l'on purifie par préparation et cristallisation d'un oxalate.

Purification par l'oxalate.

On dissout 6,21 g de produit obtenu ci-dessus et 2,93 g d'acide oxalique au reflux et sous agitation dans 140 cm3 d'isopropanol, filtre à chaud, concentre le filtrat sous pression réduite à 100 cm3 et laisse refroidir à 20°C. On essore le sel obtenu, le rince par un mélange isopropanol-éther, sèche sous pression réduite et obtient 4,24 g de produit que l'on reprend par de l'eau. On ajoute du chlorure de méthylène à la suspension obtenue, alcalinise par addition fractionnée de bicarbonate de sodium, décante et extrait par du chlorure de méthylène. On réunit les phases organiques, lave à l'eau jusqu'à neutralité, sèche, traite au charbon actif, élimine les solvants sous pression réduite. On reprend la résine obtenue par de l'éther à chaud. Après refroidissement, on essore les cristaux obtenus, sèche sous pression réduite à 20°C, puis à 60°C et obtient 3,26g de produit attendu fondant à 195°C.

Préparation de la 1,3-dihydro 2-thioxo 3,5-diméthyl 2H-1,4-benzodiazépine.

On introduit 10,2 g de pentasulfure de phosphore dans un mélange renfermant 13,33 g de 1,2-dihydro 3,5-diméthyl 2H-1,4-benzodiazépine 2-one (préparé selon le brevet français 1 326 838 ou le brevet belge 662 240), 160 cm3 de chloroforme, 13,3 cm3 d'hexaméthyl phosphotriamide, porte 4 heures au reflux, élimine le chloroforme sous pression réduite. Au résidu obtenu, on ajoute lentement 200 cm3 d'une solution saturée de bicarbonate de sodium pour amener le pH à 9-10, dilue avec 50 cm3 d'eau et agite à température ambiante sous pression réduite jusqu'à concrétion de la gomme. On essore, rince, sèche sous pression réduite, empâte au reflux le résidu dans 300 cm3 d'acétate d'éthyle, ajoute du charbon actif, porte 5 minutes au reflux, filtre à chaud et rince à chaud l'insoluble. On concentre à sec le filtrat sous pression réduite. On effectue un entraînement sous pression réduite par 20 cm3 d'éther isopropylique pour éliminer les solvants du produit cristallisé. On le reprend par 40 cm3 d'éther isopropylique, empâte, essore, rince à l'éther isopropylique, sèche sous pression réduite et obtient 7,75 g de produit.
$[\alpha]_D = -80° \pm 2,5°$ (c = 0,5% $CHCl_3$).

Exemple 2 : 4,6-diméthyl 1-(4-chlorophényl) 4H-1,2,4-triazolo [4,3-a] [1,4] benzodiazépine.

On opère de manière analogue à celle décrite à l'exemple 1, en utilisant au départ 25 g de produit de formule (II), 450 cm3 de toluène et 23 g d'hydrazide de l'acide 4-chlorobenzoïque. On maintient le reflux pendant 24 heures puis élimine le solvant sous pression réduite. On reprend le résidu à l'acétate d'éthyle, sèche et traite au charbon actif puis évapore à sec. On dissout les 50 g de produit brut obtenu dans 150 cm3 de tétrahydrofuranne, ajoute une solution de 15,6 g d'acide oxalique dans 120 cm3 de tétrahydrofuranne et maintient sous agitation. On essore les cristaux, les lave au tétrahydrofuranne puis à l'éther éthylique et les sèche. On obtient 8,4 g de produit. F = 220°C. On reprend le produit par 50 cm3 d'eau, ajoute lentement 6 g de bicarbonate de sodium, extrait au chlorure de méthylène, lave à l'eau, sèche et concentre à sec. On obtient 5,6 g de produit attendu. F = 230°C.
Analyse : $C_{19}H_{15}N_4Cl$ = 382,801.
Calculé : C% 66,98 H% 4,68 N% 17,36 Cl% 10,98
Trouvé : 67,1 4,8 17,2 10,8

Les composés cités ci-après peuvent également être obtenus selon l'invention :
- la 4,6-diméthyl 1-(3,4-diméthoxyphényl) 4H-1,2,4-triazolo [4,3-a] [1,4] benzodiazépine ;
- la 4,6-diméthyl 1-(2 ou 3-chlorophényl) 4H-1,2,4-triazolo [4,3-a] [1,4] benzodiazépine ;
- la 4,6-diméthyl 1-(2, 3 ou 4-bromophényl) 4H-1,2,4-triazolo [4,3-a] [1,4] benzodiazépine ;
- la 4,6-diméthyl 1-(2, 3 ou 4-fluorophényl) 4H-1,2,4-triazolo [4,3-a] [1,4] benzodiazépine ;
- la 4,6-diméthyl 1-(2,4 ou 2,5-dichlorophényl) 4H-1,2,4-triazolo [4,3-a] [1,4] benzodiazépine ;
- la 4,6-diméthyl 1-(2,4 ou 2,5-dibromophényl) 4H-1,2,4-triazolo [4,3-a] [1,4] benzodiazépine ;
- la 4,6-diméthyl 1-(2,4 ou 2,5-difluorophényl) 4H-1,2,4-triazolo [4,3-a] [1,4] benzodiazépine ;
- la 4,6-diméthyl 1-(3 ou 4-trifluorométhylphényl) 4H-1,2,4-triazolo [4,3-a] [1,4] benzodiazépine ;

ainsi que leurs sels d'addition avec les acides.

<u>Exemple de compositions pharmaceutiques</u>.

<u>Exemple 3</u>:

On a préparé des comprimés répondant à la formule suivante:
- Produit de l'exemple 1 50 mg
- Excipient q.s. pour un comprimé terminé à 350 mg

(Détail de l'excipient : lactose, talc, amidon, stéarate de magnésium).

<u>ETUDE PHARMACOLOGIQUE DU PRODUIT DE L'EXEMPLE 1.</u>

1. Action sur la biosynthèse des prostaglandines in vitro.

<u>Méthode</u>.

L'acide arachidonique est converti en prostaglandines (PGs) de la série 2 par une cyclo-oxygénase contenue dans une préparation microsomale de vésicules séminales de taureau effectuée selon la méthode de Tagekuchi et al. (1).

Le précurseur, à la contraception de $15 \times 10^{-6}$M, est incubé en présence d'une concentration fixe en protéines de la préparation de vésicules séminales et du produit à tester pendant 30 minutes à 37°C. Après blocage de la réaction par immersion dans l'eau bouillante pendant une minute puis centrifugation, les prostaglandines sont dosées par radio-immuno essai. Cette technique, inspirée de celle de Dray et al. (2), permet d'évaluer spécifiquement les $PGE_2$ et $PGF_2$ alpha. L'activité inhibitrice du produit ($CI_{50}$) est calculée sur la somme de ces deux prostaglandines.

La $CI_{50}$ trouvée est de $2 \times 10^{-6}$M.

2. Etude de l'activité analgésique.

Test des étirements provoqués par l'acide acétique. Le test employé est basé sur le fait signalé par R. KOSTER et Col. (Fed. Proc. 1959, <u>1B</u> 412) selon lequel l'injection intrapéritonéale d'acide acétique provoque, chez la souris, des mouvements répétés d'étirements et de torsions pouvant persister pendant plus de 6 heures. Les analgésiques préviennent ou diminuent ce syndrome qui peut être considéré comme l'extériorisation d'une douleur abdominale diffuse. L'acide acétique est administré à la dose de 100 mg/kg soit 1 ml d'une solution aqueuse à 1% par 100 g de poids corporel.

Le produit étudié est administré per os une demi-heure avant l'injection d'acide acétique, les souris étant à jeun depuis 7 heures.

Les étirements sont observés et comptés pour chaque souris pendant une période d'observation de 15 minutes.

Les résultats sont exprimés en dose active 50 ($DA_{50}$), c'est-à-dire la dose qui permet d'obtenir une diminution de 50% du nombre des étirements par rapport aux animaux témoins. La $DA_{50}$ trouvée a été de 4 mg/kg.

(1) TAGEKUCHI, C., KUHNO, E. and SIH, C.J.
Mechanism of prostaglandin biosynthesis. I characterisation and essay of bovine prostaglandin synthetase.
Biochemistry, 1971, <u>10</u>, 2372.

(2) DRAY F., CHARBONNEL B. and MACLOUF J.
Radioimmunoessay of prostaglandins F, $E_1$ and $E_2$ in human plasma.
European J. Clin. Invest., 1975, <u>5</u>, 311.

3. Etude de l'activité anti-inflammatoire.

L'activité anti-inflammatoire a été déterminée sur le test de l'arthrite provoquée par la carraghénine chez le rat.

On administre, à des rats mâles pesant de 130 à 150 g, 0,05 cm3 d'une suspension stérile à 1% de carraghénine dans l'articulation tibio-tarsienne d'une patte postérieure.

Simultanément, on administre le produit à étudier per os dans une suspension de carboxyméthylcellulose à 0,25% et de Tween à 0,02%.

Le volume de la patte est mesuré avant l'administration, puis 2 heures, 4 heures, 6 heures, 8 heures et 24 heures après.

L'intensité de l'inflammation est maxima 4 à 6 heures après l'injection de carraghénine. La différence du volume des pattes des aminaux traités et des témoins met en évidence l'action anti-inflammatoire du médicament.

On détermine la $DA_{50}$, c'est-à-dire la dose qui permet d'obtenir une diminution de l'oedème de 50%.

Pour le produit de l'exemple 1, la $DA_{50}$ a été trouvée de 50 mg/kg.

**Revendications pour les Etats contractants CH, DE, GB, IT, LI, NL**

1.- Les composés de formule (I) :

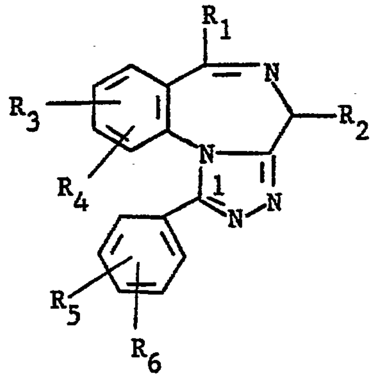

(I)

dans laquelle $R_1$ et $R_2$ représentent un atome d'hydrogène ou un radical alcoyle renfermant de 1 à 5 atomes de carbone, $R_3$, $R_4$, $R_5$, $R_6$, identiques ou différents, en position quelconque sur les noyaux benzéniques, représentent un atome d'hydrogène, un radical hydroxyle, un radical alcoyle renfermant de 1 à 4 atomes de carbone, un radical $Oalc_1$, $alc_1$ représentant un radical alcoyle renfermant de 1 à 4 atomes de carbone, un radical $NH_2$, $NHalc_2$, $alc_2$ représentant un radical alcoyle renfermant de 1 à 4 atomes de carbone, un radical

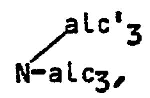

$alc_3$ et $alc'_3$, identiques ou différents, représentant un radical alcoyle renfermant de 1 à 4 atomes de carbone, un atome d'halogène, un radical $CF_3$ ou un radical $NO_2$, à la condition que $R_5$ et $R_6$ ne représentent pas à la fois un atome d'hydrogène, sous toutes les formes racémiques ou optiquement actives, ainsi que leurs sels d'addition avec les acides.

2.- Les composés de formule (I) tels que définis à la revendication 1, pour lesquels $R_1$ et $R_2$ représentent un radical méthyle, $R_3$ et $R_4$ représentent un atome d'hydrogène, et soit $R_5$ représente un atome d'hydrogène et $R_6$ représente un radical méthoxy, un atome d'halogène ou un radical $CF_3$, soit $R_5$ et $R_6$, identiques, représentent chacun un radical méthoxy, un atome d'halogène ou un radical $CF_3$, sous leurs formes racémiques ou optiquement actives, ainsi que leurs sels d'addition avec les acides.

3.- La 4,6-diméthyl 1-(4-méthoxyphényl) 4H-1,2,4-triazolo [4,3-a] [1,4] benzodiazépine, ainsi que la 4,6-diméthyl 1-(4-chlorophényl 4H-1,2,4-triazolo [4,3-a] [1,4] benzodiazépine, sous leurs formes racémique ou optiquement actives, ainsi que leurs sels d'addition avec les acides.

4.- Procédé de préparation des composés de formule (I), tels que définis à la revendication 1, caractérisé en ce que l'on soumet un composé de formule II :

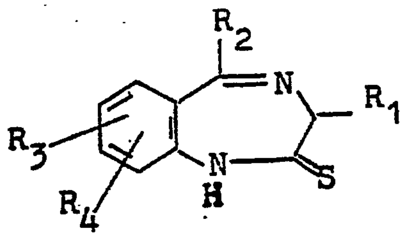

(II)

dans laquelle $R_1$, $R_2$, $R_3$ et $R_4$ ont les significations précédentes, sous forme racémique ou optiquement active, à l'action d'un composé de formule (III) :

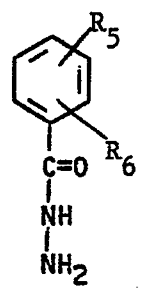

(III)

dans laquelle $R_5$ et $R_6$ ont les significations précédentes, pour obtenir un composé de formule (I) sous forme racémique ou optiquement active, que l'on transforme, si désiré, en sels par action d'un acide.

5.- A titre de médicaments, les composés de formule (I), tels que définis aux revendications 1 et 2, sous formes racémiques ou optiquement actives, ainsi que leurs sels d'addition avec les acides pharmaceutiquement acceptables.

6.- A titre de médicaments, les composés définis à la revendication 3, sous leurs formes racémique ou optiquement actives, ainsi que leurs sels d'addition avec les acides pharmaceutiquement acceptables.

7.- Les compositions pharmaceutiques renfermant à titre de principe actif, l'un au moins des médicaments tels que définis aux revendications 5 et 6.

**Revendications pour l'Etat contractant ES**

1.- Procédé de préparation des composés de formule (I) :

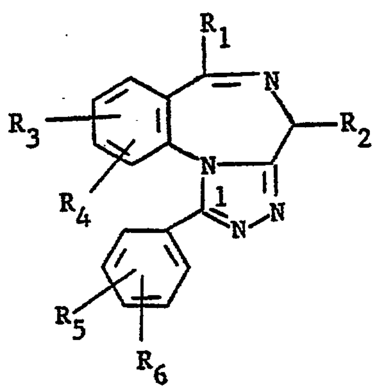

(I)

dans laquelle $R_1$ et $R_2$ représentent un atome d'hydrogène ou un radical alcoyle renfermant de 1 à 5 atomes de carbone, $R_3$, $R_4$, $R_5$, $R_6$, identiques ou différents, en position quelconque sur les noyaux benzéniques, représentent un atome d'hydrogène, un radical hydroxyle, un radical alcoyle renfermant de 1 à 4 atomes de carbone, un radical $Oalc_1$, $alc_1$ représentant un radical alcoyle renfermant de 1 à 4 atomes de carbone, un radical $NH_2$, $NHalc_2$, $alc_2$ représentant un radical alcoyle renfermant de 1 à 4 atomes de carbone, un radical

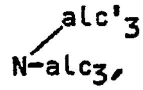

alc3 et alc'3, identiques ou différents, représentant un radical alcoyle renfermant de 1 à 4 atomes de carbone, un atome d'halogène, un radical $CF_3$ ou un radical $NO_2$, à la condition que $R_5$ et $R_6$ ne représentent pas à la fois un atome d'hydrogène, sous toutes les formes racémiques ou optiquement actives, ainsi que de leurs sels d'addition avec les acides, caractérisé en ce que l'on soumet un composé de formule II :

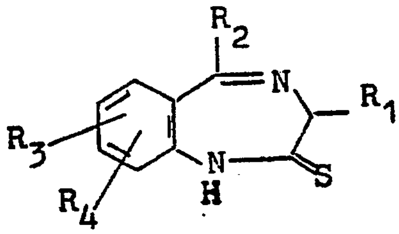

(II)

dans laquelle $R_1$, $R_2$, $R_3$ et $R_4$ ont les significations précédentes, sous forme racémique ou optiquement active, à l'action d'un composé de formule (III) :

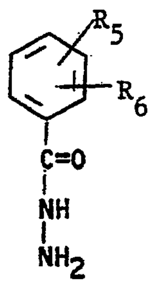

(III)

dans laquelle $R_5$ et $R_6$ ont les significations précédentes, pour obtenir un composé de formule (I) sous forme racémique ou optiquement active, que l'on transforme, si désiré, en sels par action d'un acide.

2.- Procédé selon la revendication 1, caractérisé en ce que l'on utilise au départ un composé de formule (II) dans laquelle $R_1$ et $R_2$ représentent un radical méthyle et $R_3$ et $R_4$ représentent un atome d'hydrogène, ainsi qu'un composé de formule (II) dans laquelle soit $R_5$ représente un atome d'hydrogène et $R_6$ représente un radical méthoxy, un atome d'halogène ou un radical $CF_3$, soit $R_5$ et $R_6$, identiques, représentent un radical méthoxy, un atome d'halogène ou un radical $CF_3$.

3.- Procédé selon la revendication 1 ou 2, caractérisé en ce que l'on utilise au départ un composé de formule (II) dans laquelle $R_1$ et $R_2$ représentent un radical méthyle, $R_3$ et $R_4$ représenent un atome d'hydrogène, ainsi qu'un composé de formule (III) dans laquelle $R_5$ représente un atome d'hydrogène et $R_6$ représente un radical 4-chloro.

**Patentansprüche für die Vertragsstaaten CH, DE, GB, IT, LI, NL**

1. Verbindungen der Formel (I)

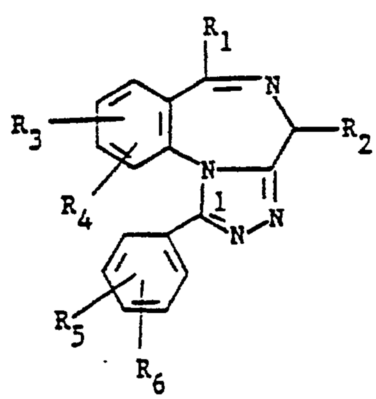

(I)

worin $R_1$ und $R_2$ für ein Wasserstoffatom oder einen Alkylrest mit 1 bis 5 Kohlenstoffatomen stehen, $R_3$, $R_4$, $R_5$ und $R_6$, die gleich oder voneinander verschieden sind, in beliebiger Stellung an den Benzolringen ein Wasserstoffatom, einen Hydroxylrest, eine Alkylgruppe mit 1 bis 4 Kohlenstoffatomen, einen Rest $Oalc_1$, worin $alc_1$ einen Alkylrest mit 1 bis 4 Kohlenstoffatomen darstellt, einen Rest $NH_2$, $NHalc_2$, worin $alc_2$ einen Alkylrest mit 1 bis 4 Kohlenstoffatomen wiedergibt, einen Rest

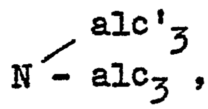 ,

worin $alc_3$ und $alc'_3$, die gleich oder voneinander verschieden sind, einen Alkylrest mit 1 bis 4 Kohlenstoffatomen darstellen, ein Halogenatom, einen Rest $CF_3$ oder einen Rest $NO_2$ bedeuten, mit der Maßgabe, daß $R_5$ und $R_6$ nicht gleichzeitig ein Wasserstoffatom wiedergeben, in sämtlichen ihrer racemischen oder optisch aktiven Formen sowie deren Additionssalze mit Säuren.

2. Verbindungen der Formel (I) gemäß Anspruch 1, worin $R_1$ und $R_2$ für eine Methylgruppe stehen, $R_3$ und $R_4$ ein Wasserstoffatom bedeuten und entweder $R_5$ für ein Wasserstoffatom steht und $R_6$ für eine Methoxygruppe, ein Halogenatom oder einen Rest $CF_3$ steht oder $R_5$ und $R_6$, die identisch sind, jeweils eine Methoxygruppe, ein Halogenatom oder eine Gruppe $CF_3$ wiedergeben, in ihren racemischen oder optisch aktiven Formen sowie deren Additionssalze mit Säuren.

3. 4,6-Dimethyl-1-(4-methoxyphenyl)-4H-1,2,4-triazolo[4,3-a]-[1,4]-benzodiazepin sowie 4,6-Dimethyl-1(4-chlorphenyl)-4H-1,2,4-triazolo[4,3-a]-[1,4]-benzodiazepin in ihren racemischen oder optisch aktiven Formen sowie deren Additionssalze mit Säuren.

4. Verfahren zur Herstellung der Verbindungen der Formel (I) gemäß Anspruch 1, dadurch gekennzeichnet, daß man eine Verbindung der Formel II

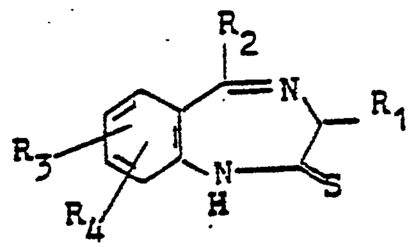

(II)

worin $R_1$, $R_2$, $R_3$ und $R_4$ die vorstehenden Bedeutungen besitzen, in racemischer oder optisch aktiver Form der Einwirkung einer Verbindung der Formel III

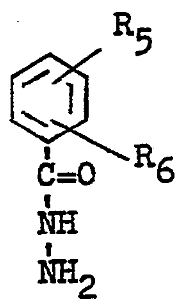

(III)

worin $R_5$ und $R_6$ die vorstehenden Bedeutungen besitzen, unterzieht, um zu einer Verbindung der Formel (I) in racemischer oder optisch aktiver Form zu gelangen, die man gewünschtenfalls durch Umsetzung mit einer Säure in Salze überführt.

5. Als Arzneimittel die Verbindungen der Formel (I) gemäß den Ansprüchen 1 und 2 in racemischer oder optisch aktiver Form sowie deren Additionssalze mit pharmazeutisch verträglichen Säuren.

6. Als Arzneimittel die Verbindungen gemäß Anspruch 3 in racemischer oder optisch aktiver Form sowie deren Additionssalze mit pharmazeutisch verträglichen Säuren.

7. Pharmazeutische Zusammensetzungen, enthaltend als Wirkstoff mindestens eines der Arzneimittel gemäß den Ansprüchen 5 und 6.

**Patentansprüche für den Vertragsstaat ES**

1. Verfahren zur Herstellung der Verbindungen der Formel (I)

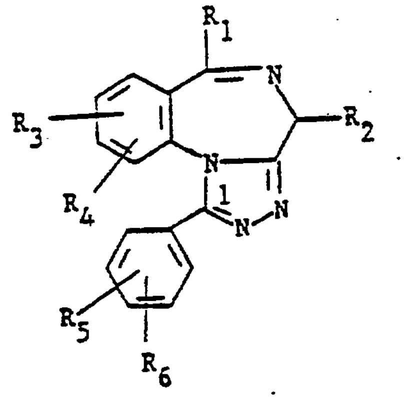

(I)

worin $R_1$ und $R_2$ für ein Wasserstoffatom oder einen Alkylrest mit 1 bis 5 Kohlenstoffatomen stehen, $R_3$, $R_4$, $R_5$ und R6, die gleich oder voneinander verschieden sind, in beliebiger Stellung an den Benzolringen ein Wasserstoffatom, einen Hydroxylrest, eine Alkylgruppe mit 1 bis 4 Kohlenatomen, einen Rest $Oalc_1$, worin $alc_1$ einen Alkylrest mit 1 bis 4 Kohlenstoffatomen darstellt, einen Rest $NH_2$, $NHalc_2$, worin $alc_2$ einen Alkylrest mit 1 bis 4 Kohlenstoffatomen wiedergibt, einen Rest

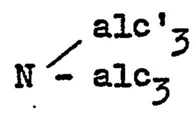 ,

worin $alc_3$ und $alc'_3$, die gleich oder voneinander verschieden sind, einen Alkylrest mit 1 bis 4 Kohlenstoffatomen darstellen, ein Halogenatom, einen Rest $CF_3$ oder einen Rest $NO_2$ bedeuten, mit der Maßgabe, daß $R_5$ und $R_6$ nicht gleichzeitig ein Wasserstoffatom wiedergeben, in sämtlichen ihrer racemischen oder optisch aktiven Formen sowie von deren Additionssalzen mit Säuren, dadurch gekennzeichnet, daß man eine Verbindung der Formel II

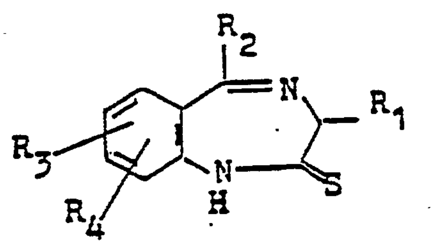

(II)

worin $R_1$, $R_2$, $R_3$ und $R_4$ die vorstehenden Bedeutungen besitzen, in racemischer oder optisch aktiver Form der Einwirkung einer Verbindung der Formel (III)

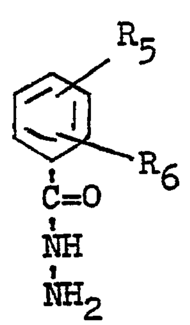

(III)

worin $R_5$ uad $R_6$ die vorstehenden Bedeutungen besitzen, unterzieht, um zu einer Verbindung der Formel (I) in racemischer oder optisch aktiver Form zu gelangen, die man gewünschtenfalls durch Umsetzung mit einer Säure in Salze überführt.

2. Verfahren gemäß Anspruch 1, dadurch gekennzeichnet, daß man von einer Verbindung der Formel (II) ausgeht, worin $R_1$ und $R_2$ für eine Methylgrppe stehen und $R_3$ und $R_4$ ein Wasserstoffatom bedeuten, sowie von einer Verbindung der Formel (II), worin entweder $R_5$ ein Wasserstoffatom bedeutet und

$R_6$ eine Methoxygruppe, ein Halogenatom oder einen Rest $CF_3$ wiedergibt oder $R_5$ und $R_6$, die identisch sind, für eine Methoxygruppe, ein Halogenatom oder einen Rest $CF_3$ stehen.

3. Verfahren gemäß Anspruch 1 oder 2, dadurch gekennzeichnet, daß man von einer Verbindung der Formel (II) ausgeht, worin $R_1$ und $R_2$ für eine Methylgruppe stehen, $R_3$ und $R_4$ ein Wasserstoffatom bedeuten, sowie von einer Verbindung der Formel (III), worin $R_5$ für ein Wasserstoffatom steht und $R_6$ für einen 4-Chloro-Rest steht.

**Claims for the contracting States CH, DE, GB, IT, LI, NL**

1. The compounds of formula (I):

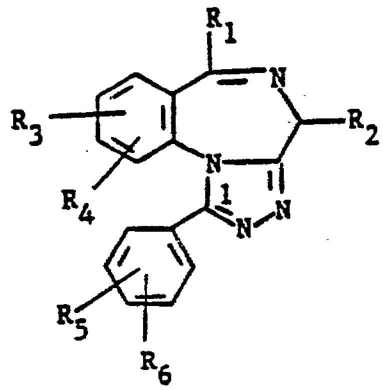

(I)

in which $R_1$ and $R_2$ represent a hydrogen atom or an alkyl radical containing from 1 to 5 carbon atoms, $R_3$, $R_4$, $R_5$, $R_6$, identical or different, in any position on the benzene nuclei, represent a hydrogen atom, a hydroxyl radical, an alkyl radical containing from 1 to 4 carbon atoms, an $Oalk_1$ radical, $alk_1$ representing an alkyl radical containing from 1 to 4 carbon atoms, an $NH_2$, $NHalk_2$ radical, $alk_2$ representing an alkyl radical containing from 1 to 4 carbon atoms, an

$$N(alk_3)(alk'_3)$$

radical, $alk_3$ and $alk'_3$, identical or different representing an alkyl radical containing from 1 to 4 carbon atoms, a halogen atom, a $CF_3$ or an $NO_2$ radical, on the condition that $R_5$ and $R_6$ does not represent at the same time a hydrogen atom, in all the racemic or optically active forms, as well as their addition salts with acids.

2. The compounds of formula (I) as defined in claim 1, for which $R_1$ and $R_2$ represent a methyl radical, $R_3$ and $R_4$ represent a hydrogen atom, and either $R_5$ represents a hydrogen atom and $R_6$ represents a methoxy radical, a halogen atom or a $CF_3$ radical, or $R_5$ and $R_6$, identical, each represent a methoxy radical, a halogen atom or a $CF_3$radical, in all their racemic or optically active forms, as well as their addition salts with acids.

3. 4,6-dimethyl-1-(4-methoxyphenyl)-4H-1,2,4-triazolo-[4,3-a]-[1,4]-benzodiazepine, as well as 4,6-dimethyl-1-(4-chlorophenyl-4H-1,2,4-triazolo-[4,3-a]-[1,4]-benzodiazepine, in all their racemic or optically active forms, as well as their addition salts with acids.

4. Preparation process for compounds of formula (I), as defined in claim 1, characterised in that a compound of formula II:

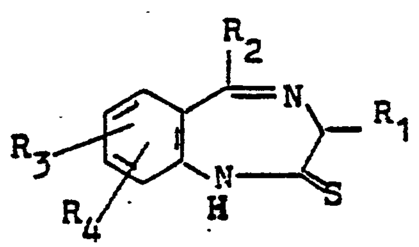

(II)

in which $R_1$, $R_2$, $R_3$ and $R_4$ have the previous meanings, in the racemic or optically active form, is subjected to the action of a compound of formula (III):

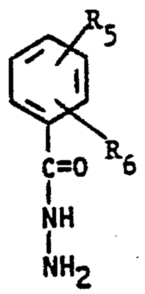

(III)

in which $R_5$ and $R_6$ have the previous meanings, so as to obtain a compound of formula (I) in the racemic or optically active form, which is converted, if desired, into salts by the action of an acid.

5. As medicaments, the compounds of formula (I), as defined in claims 1 and 2, in the racemic or optically active forms, as well as their addition salts with pharmaceutically acceptable acids.

6. As medicaments, the compounds defined in claim 3, in their racemic or optically active forms, as well as their addition salts with pharmaceutically acceptable acids.

7. The pharmaceutical compositions containing as active principle, at least one of the medicaments as defined in claims 5 and 6.

**Claims for the contracting State ES**

1. Preparation process for compounds of formula (I):

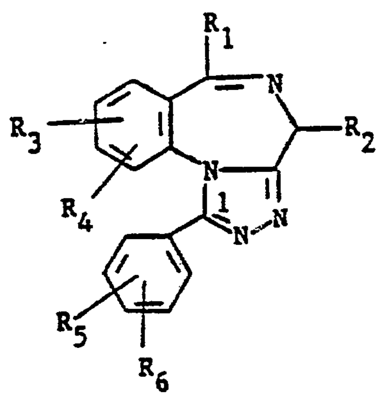

(I)

in which $R_1$ and $R_2$ represent a hydrogen atom or an alkyl radical containing from 1 to 5 carbon atoms, $R_3$, $R_4$, $R_5$, $R_6$, identical or different, in any position on the benzene nuclei, represent a hydrogen atom, a hydroxyl radical, an alkyl radical containing from 1 to 4 carbon atoms, an $Oalk_1$ radical, $alk_1$ representing an alkyl radical containing from 1 to 4 carbon atoms, an $NH_2$, $NHalk_2$ radical, $alk_2$ representing an alkyl radical containing from 1 to 4 carbon atoms,

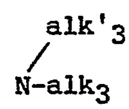

radical, $alk_3$ and $alk'_3$, identical or different, representing an alkyl radical containing from 1 to 4 carbon atoms, a halogen atom, a $CF_3$ radical or an $NO_2$ radical, on the condition that $R_5$ and $R_6$ do not represent at the same time a hydrogen atom, in all racemic or optically active forms, as well as their addition salts with acids, characterised in that a compound of formula II:

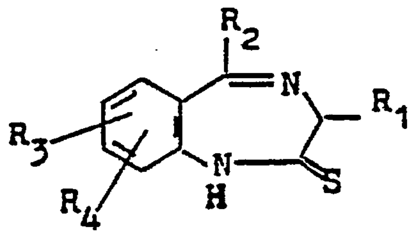

(II)

in which $R_1$, $R_2$, $R_3$ and $R_4$ have the previous meanings, in the racemic or optically active form, is subjected to the action of a compound of formula (III):

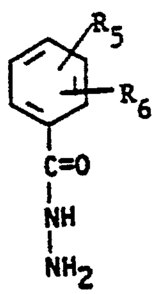

(III)

in which $R_5$ and $R_6$ have the previous meanings, so as to obtain a compound of formula (I) in the racemic or optically active form, which is converted, if desired, into salts by the action of an acid.

2. Process according to claim 1, characterised in that to start with a compound of formula (II) is used, in which $R_1$ and $R_2$ represent a methyl radical and $R_3$ and $R_4$ represent a hydrogen atom, as well as a compound of formula (III) in which either $R_5$ represents a hydrogen atom and $R_6$ represents a methoxy radical, a halogen atom or a $CF_3$ radical, or $R_5$ and $R_6$, identical, represent a methoxy radical, a halogen atom or a $CF_3$ radical.

3. Process according to claim 1 or 2, characterised in that to start with a compound of formula (II) is used, in which $R_1$ and $R_2$ represent a methyl radical, $R_3$ and $R_4$ represent a hydrogen atom, as well as a compound of formula (III) in which $R_5$ represents a hydrogen atom and $R_6$ represents a 4-chloro radical.